# EUROPÄISCHE PATENTANMELDUNG

(11) **EP 1 447 101 A1**
(43) Veröffentlichungstag der Anmeldung: **18.08.2004**
(21) Anmeldenummer: 04003362.3
(22) Anmeldetag: 16.02.2004
(51) Int. Cl.: A61L 2/02, A61L 2/04, A61K 9/14

(54) **Verfahren zur Herstellung von sterilen pharmazeutischen Wirkstoffen**

(30) Priorität: 17.02.2003 DE 10306761
(71) Anmelder: Völkl, Klaus-Peter, Dr., 39112 Magdeburg (DE)
(72) Erfinder: Völkl, Klaus-Peter, Dr., 39112 Magdeburg (DE)
(74) Vertreter: Neuhäuser, Uwe

(57) **Zusammenfassung**

Die Aufgabe, ein verbessertes Verfahren zur Herstellung von sterilen pharmazeutischen Wirkstoffen anzugeben, mit welchem bei geringerem Aufwand an Zeit und Energie sowie geringerem Lösungsmitteleinsatz ein erhöhter Durchsatz pro Zeiteinheit erzielbar ist, wird erfindungsgemäß durch eine Kombination aus waschen und sterilfiltrieren gelöst.

## Beschreibung

Die Erfindung betrifft ein Verfahren zur Herstellung von sterilen pharmazeutischen Wirkstoffen, wie Cytostatika, Steroiden, Antibiotika o. a., wobei die zu sterilisierenden Wirkstoffe zunächst pulverförmig in ein Lösungsmittel eingebracht werden und anschließend die sich ausbildende Lösung einer Sterilfiltration unterzogen und nachfolgend der sterile Wirkstoff (Cytostatikum, Steroid, Antibiotikum o. a.) vom Lösungsmittel separiert respektive getrocknet wird.

Eine Reihe von pharmazeutisch genutzten Stoffen, wozu auch Cytostatika, Steroide und Antibiotika gehören, müssen in steriler Form, d. h., befreit von jeglichen Verunreinigungen, auch mikrobieller Herkunft, vorliegen.

Verfahren zur Herstellung und Reinigung eines Antibiotikums, wie beispielsweise Ampicillin, sind seit langem bekannt (DE 16 45 980 A1, DE 21 42 180 A1, DE 32 08 506 A1 ).

Mit der DE 16 45 980 A1 wird danach vorgeschlagen, rohes Ampicillin in einem geeigneten wassermischbaren Lösungsmittel unter Zugabe eines Lösungsvermittlers, wie einer Säure, zu lösen, diese Lösung zu filtrieren, um eine völlig klare Lösung zu erhalten, nachfolgend das Ampicillin durch Zugabe einer geeigneten Base auszufällen, anschließend den sich ergebenden Niederschlag abzufiltrieren und in mehreren Verfahrensschritten zu waschen und schlußendlich den ausgepreßten Filterkuchen zu trocknen. Aufgrund der Tatsache, dass lediglich Sättigungsgrade von maximal 20 % erreichbar sind, ist für einen Fachmann leicht nachvollziehbar, dass nach diesem Verfahren ein erheblicher Kostenaufwand, resultierend aus erhöhtem Lösungsmittelverbrauch und erhöhtem Zeitaufwand, zu verzeichnen ist.
Sicherlich kann durch eine bestimmte Temperaturführung des Verfahrensprozesses, wie sie in den Druckschriften DE 21 42 180 A1 und DE 32 08 506 A1 näher offenbart ist, der Sättigungsgrad in Grenzen gesteigert werden, jedoch resultieren daraus weitere Aufwendungen, die sich insbesondere durch einen erhöhten Energieverbrauch darstellen.
Hier setzt die nachfolgend beschrieben Erfindung an.

Aufgabe der Erfindung ist es, ein verbessertes Verfahren zur Herstellung von sterilen pharmazeutischen Wirkstoffen, wie Cytostatika, Steroiden, Antibiotika o. a. anzugeben, mit welchem im Hinblick auf den eingangs geschilderten Stand der Technik bei geringerem Aufwand an Zeit und Energie sowie geringerem Lösungsmitteleinsatz ein erhöhter Durchsatz erzielbar ist.

Ausgehend von einem Verfahren zur Herstellung von sterilen pharmazeutischen Wirkstoffen, wie Cytostatika, Steroiden, Antibiotika o. a., nach welchem der jeweilige Wirkstoff zunächst pulverförmig in ein Lösungsmittel eingebracht, anschließend die sich ausbildende Lösung einer Sterilfiltration unterzogen und nachfolgend der sterile Wirkstoff vom Lösungsmittel separiert respektive getrocknet wird, wird die Aufgabe erfindungsgemäß dadurch gelöst, dass
- die Partikel des Wirkstoffes derart im Lösungsmittel gewaschen respektive von Verunreinigungen befreit und sterilisiert werden, dass sich eine äußere Schicht der Partikel samt besagter Verunreinigungen auch mikrobieller Herkunft ablöst und durch das Lösungsmittel aufgenommen und abtransportiert wird,
- ungelöste, jedoch gereinigte Partikelanteile sich als Sediment absetzen,
- das mit den Verunreinigungen und gelösten Partikelanteilen behaftete Lösungsmittel der Sterilfiltration zugeführt wird,
- nachfolgend das gewonnene Sediment aus gereinigten, sterilen Partikelanteilen mit dem sterilisierten Filtrat vereint und diese Vereinigung in einem Reaktionsgefäß schließlich in Lösungsmittel und sterilem Wirkstoff getrennt wird.

Gemäß einer besonders vorteilhaften Weiterbildung der Erfindung ist vorgesehen, dass die Partikel des Wirkstoffes während des Waschvorgangs vom Lösungsmittel gegen die Gravitation und/oder gegen eine Zentrifugalkraft durchströmt werden.

Des Weiteren wird vorgeschlagen, dass die gereinigten, sterilen Partikelanteile des Sediments samt dem sterilisierten Filtrat vermittels Temperaturveränderung getrocknet werden.

Wie die Erfindung noch vorsieht, können sowohl wäßrige als auch organische Lösungsmittel zur Anwendung kommen.

Weiterhin wird im Sinne der Erfindung vorgeschlagen, dass das durch Trocknung vom Sediment samt sterilem Filtrat separierte Lösungsmittel wieder dem Verfahrensprozeß zugeführt wird.

Ferner zeichnet sich die Erfindung durch ein Arzneimittel aus, welches sterile pharmazeutische Wirkstoffe, wie Cytostatika, Steroide, Antibiotika o. a., enthält, die ihrerseits nach dem erfindungsgemäßen Verfahren hergestellt sind.

Das vorgeschlagene Verfahren hat im Hinblick auf herkömmliche den wesentlichen Vorteil, dass trotz relativ geringer Sättigungsgrade im Lösungsmittel von zum Teil nur 20g/| ≙ 2 % ein höherer Durchsatz pro Zeiteinheit zu verzeichnen ist. Ein vollständiges Lösen des Wirkstoffes im verwendeten Lösungsmittel zur Sterilisation desselben ist nicht erforderlich, denn im Rahmen umfangreicher Versuche wurde gefunden, dass es ausreicht, lediglich die äußere Schicht der Partikel vermittels Waschung zu lösen, da hier die Verunreinigungen anhaften und durch das Lösungsmittel aufgenommen und abtransportiert werden können.

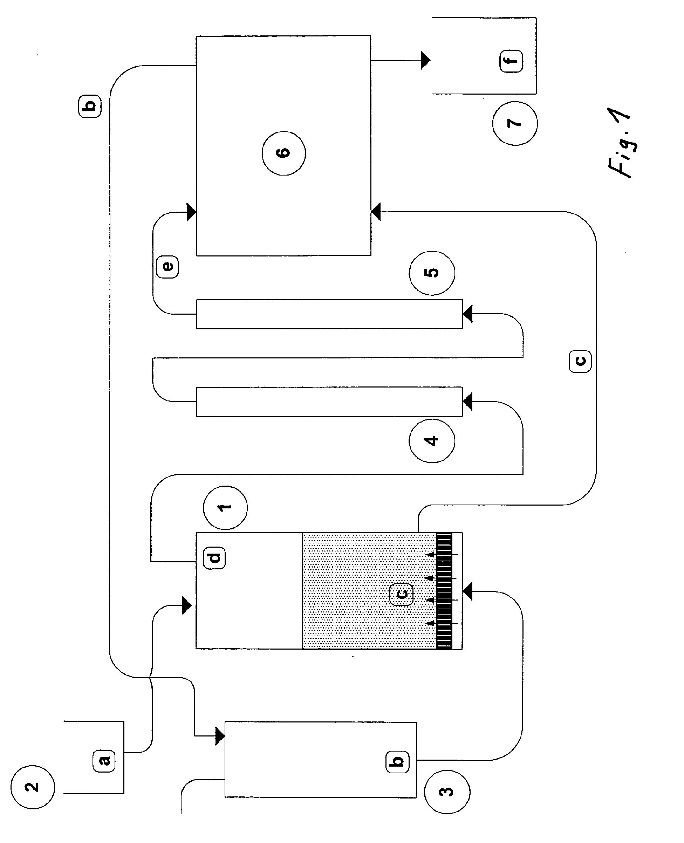

Die Erfindung wird nachstehend anhand eines in der Zeichnung schematisch dargestellten Flussbildes näher erläutert.

Danach wird zunächst in einem Reaktionsgefäß "Waschen" **1** eine Suspension aus einem pulverförmig (kristalliner und/oder amorpher Zustand) bereitgestellten pharmazeutischen Wirkstoff **a** (unsteril) und einem Lösungsmittel **b** erzeugt. Als Lösungsmittel **b** können sowohl an sich bekannte wäßrige als auch organische Lösungsmittel **b** zur Anwendung kommen.
Wirkstoff **a** und Lösungsmittel **b** werden jeweils aus einem Vorratsgefäß 2 bzw. **3** dem Reaktionsgefäß **1** zugeführt.

Nachfolgend werden die Partikel des Wirkstoffes **a** im Reaktionsgefäß **1** derart gewaschen respektive von Verunreinigungen befreit und sterilisiert, dass sich eine äußere Schicht der Partikel samt besagter Verunreinigungen, welche auch mikrobieller Herkunft sein können, ablöst und durch das Lösungsmittel **b** aufgenommen wird.

Im Ergebnis der bereits oben erwähnten Versuche hat es sich dabei als besonders vorteilhaft herausgestellt, den Wirkstoff a während des Waschvorgangs vom Lösungsmittel b gegen die Gravitation und/oder gegen eine nicht näher dargestellte Zentrifugalkraft durchströmen zu lassen.
Die Dauer des vorstehenden Waschvorganges wird in Abhängigkeit von der verwendeten Menge an Wirkstoff **a** und einem geeigneten Volumen an Lösungsmittel **b** gewählt.

Nach Einstellung des Waschvorgangs bzw. der Durchströmung der Partikel setzen sich ungelöste, jedoch gereinigte (sterile) Partikelanteile des Wirkstoffes **a** auf dem Boden des Reaktionsgefäßes **1** respektive vorliegend auf einer nicht näher bezeichneten Filterplatte desselben als Sediment **c** ab.

Das mit den Verunreinigungen und gelösten Partikelanteilen behaftete Lösungsmittel **d** wird dagegen vermittels einer an sich bekannten und nicht näher dargestellten Pumpe einer Sterilfiltration, vorliegend bestehend aus einem 5µm-Vorfilter **4** und einem nachgeordneten für die Sterilfiltration geeigneten 0,2µm-Filter **5**, zugeführt.

Im Anschluß daran kann das sterilisierte Filtrat **e** direkt mit den sich im Reaktionsgefäß **1** als Sediment c vorliegenden sterilen Partikelanteilen vereint und diese Vereinigung einem Reaktionsgefäß "Trennen" **6** der Partikelanteile zugeführt werden, wobei die Trennung des sterilen Wirkstoffes **f** vom verwendeten Lösungsmittels **b** vorzugsweise durch Trocknung erfolgt.

Die Trocknung der sich ergebenden Vereinigung aus dem sterilen Sediment **c** und dem sterilisierten Filtrat **e** kann bekanntermaßen vermittels Temperaturveränderung, d. h., vermittels Wärme oder Gefriertrocknung im besagten Reaktionsgefäß "Trennen" **6** durchgeführt werden.
Das separierte Lösungsmittel **b** kann ferner dem Verfahrensprozeß wieder zugeführt werden.

Abschließend wird der sterile pharmazeutische Wirkstoff **f** einem geeigneten Vorratsgefäß **7** zugeführt.

## Patentansprüche

1. Verfahren zur Herstellung von sterilen pharmazeutischen Wirkstoffen, wie Cytostatika, Steroide, Antibiotika o. a., wobei zunächst der pulverförmig bereitgestellte Wirkstoff in ein Lösungsmittel eingebracht, anschließend die sich ausbildende Lösung einer Sterilfiltration unterzogen und nachfolgend der sterile Wirkstoff vom Lösungsmittel separiert respektive getrocknet wird,
**dadurch gekennzeichnet, dass**
- die Partikel des Wirkstoffes **(a)** derart im Lösungsmittel **(b)** gewaschen respektive von Verunreinigungen befreit und sterilisiert werden, dass sich eine äußere Schicht der Partikel samt besagter Verunreinigungen auch mikrobieller Herkunft ablöst und durch das Lösungsmittel **(b)** aufgenommen und abtransportiert wird,
- ungelöste, jedoch gereinigte Partikelanteile sich als Sediment **(c)** absetzen,
- das mit den Verunreinigungen und gelösten Partikelanteilen behaftete Lösungsmittel **(d)** der Sterilfiltration **(4; 5)** zugeführt wird,
- das gewonnene Sediment **(c)** aus gereinigten, sterilen Partikelanteilen mit dem sterilisierten Filtrat **(e)** vereint und diese Vereinigung schließlich in Lösungsmittel **(b)** und sterilem Wirkstoff **(f)** getrennt wird.

2. Verfahren nach Anspruch 1,
**dadurch gekennzeichnet, dass**
die Partikel des Wirkstoffes **(a)** während des Waschvorgangs vom Lösungsmittel **(b)** gegen die Gravitation und/oder gegen eine Zentrifugalkraft durchströmt werden.

3. Verfahren nach einem der Ansprüche 1 und 2,
**dadurch gekennzeichnet, dass**
die gereinigten, sterilen Partikelanteile des Sediments **(c)** samt dem sterilisierten Filtrat **(e)** vermittels Temperaturveränderung getrocknet werden.

4. Verfahren nach einem der Ansprüche 1 bis 3,
**dadurch gekennzeichnet, dass**
wäßrige oder organische Lösungsmittel **(b)** verwendet werden.

5. Verfahren nach einem der Ansprüche 1 bis 4,
**dadurch gekennzeichnet, dass**
das durch Trocknung vom Sediment **(c)** samt sterilisiertem Filtrat **(e)** separierte Lösungsmittel **(b)** wieder dem Verfahrensprozeß zugeführt wird.

6. Arzneimittel, enthaltend sterile pharmazeutische Wirkstoffe **(f),** wie Cytostatika, Steroide, Antibiotika o. a., die ihrerseits nach einem Verfahren gemäß einem oder mehreren der Ansprüche 1 bis 5 hergestellt sind.
